Europäisches Patentamt

European Patent Office

Office européen des brevets

(19)

(11) **EP 0 861 656 A1**

(12) **EUROPÄISCHE PATENTANMELDUNG**

(43) Veröffentlichungstag:
02.09.1998 Patentblatt 1998/36

(51) Int. Cl.⁶: **A61K 7/135**

(21) Anmeldenummer: 97118999.8

(22) Anmeldetag: 31.10.1997

(84) Benannte Vertragsstaaten:
AT BE CH DE DK ES FI FR GB GR IE IT LI LU MC NL PT SE

(30) Priorität: 01.02.1997 DE 19703802

(71) Anmelder:
**Wella Aktiengesellschaft**
**64274 Darmstadt (DE)**

(72) Erfinder:
• **Lauscher, Dirk**
  **64372 Ober-Ramstadt (DE)**
• **Göttmann, Holger**
  **64395 Brensbach (DE)**

(54) **Mittel zum Bleichen der Haare**

(57) Mittel zum Entfärben oder Blondieren von Keratinfasern, insbesondere menschlichen Haaren, welches a) mindestens ein Erdalkaliperoxid und b) mindestens eine organische oder anorganische Säure enthält, sowie Verfahren zum Entfärben oder Blondieren von Keratinfasern unter Verwendung dieses Mittels.

EP 0 861 656 A1

Printed by Xerox (UK) Business Services
2.16.3/3.4

**Beschreibung**

Gegenstand der vorliegenden Erfindung ist ein Mittel zum Entfärben (Bleichen) oder Blondieren von Keratinfasern, insbesondere menschlichen Haaren, das mindestens ein Erdalkaliperoxid, mindestens eine anorganische oder organische Säure beziehungsweise deren Salze, sowie gegebenenfalls ein oder mehrere Persulfate enthält.

Zum Entfärben oder Blondieren von Haaren werden üblicherweise oxidierende Zubereitungen verwendet, welche durch Auflösen eines Blondierpulvers oder einer Blondierpaste (enthaltend anorganische Persalze, beispielsweise Ammonium-, Natrium- oder Kaliumperoxodisulfat, Alkalisalze sowie gegebenenfalls Verdickungsmittel) in einer wäßrigen Wasserstoffperoxidlösung erhalten werden. Solche Blondierrezepturen sind in der einschlägigen Fachliteratur, beispielsweise bei K. Schrader, Grundlagen und Rezepturen der Kosmetika (Hüthig Verlag, Heidelberg, 1. Aufl. 1979, 2. Aufl. 1989), C. Zviak, The Science of Hair Care (Marcel Dekker, New York, 1986) und W. Umbach, Kosmetik (Thieme Verlag, Stuttgart, 2. Aufl. 1995) beschrieben.

Der Nachteil dieser Mittel ist die hohe Konzentration an Wasserstoffperoxid zu Beginn der Anwendung, wodurch Schädigungen der Haarstruktur hervorgerufen werden können.

Aus der WO 94/16672 und den darin zitierten Quellen sind Blondiermittelzubereitungen bekannt, bei denen ein Vermischen mit einer Wasserstoffperoxidlösung nicht notwendig ist, da das Bleichpulver Natriumpercarbonat (ein Wasserstoffperoxidaddukt an Natriumcarbonat) enthält, welches bei Zugabe von Wasser Wasserstoffperoxid freisetzt. Die Wasserstoffperoxidkonzentration in dem Blondiermittel wird hierbei über die Löslichkeit des Percarbonats reguliert. Problematisch bei der Verwendung von derartigen Wasserstoffperoxidaddukten ist jedoch ihre geringe Stabilität und ihr mehr oder weniger hygroskopisches Verhalten.

Es bestand daher die Aufgabe, ein Mittel zum Entfärben oder Blondieren von Keratinfasern, insbesondere menschlichen Haaren, zur Verfügung zu stellen, das die vorgenannten Nachteile nicht aufweist und eine schonende und gleichmäßige Entfärbung oder Blondierung der Haare ermöglicht.

Überraschend wurde nun gefunden, daß die vorstehend beschriebenen Nachteile vermieden werden können, wenn als Bleichmittel eine Zubereitung verwendet wird, die Erdalkaliperoxide sowie organische oder anorganische Säuren enthält.

Gegenstand der vorliegenden Erfindung ist daher ein Mittel zum Entfärben oder Blondieren von Keratinfasern (nachfolgend „Bleichmittel" genannt), insbesondere menschlichen Haaren, welches a) mindestens ein Erdalkaliperoxid und b) mindestens eine organische oder anorganische Säure enthält.

Als organische Säure können beispielsweise genannt werden: Monocarbonsäuren wie Essigsäure, Propionsäure oder Palmitinsäure; Dicarbonsäuren wie Oxalsäure, Malonsäure oder Phthalsäure; Aminosäuren wie Glycin, Alanin oder Leucin; Aminosäuren mit weiteren funktionellen Gruppen wie Serin, Cystein, Lysin, Arginin, Ornithin, Citrullin oder Carnitin; Aminopolycarbonsäuren wie Nitrilotriessigsäure, Methylglycindiessigsäure, Ethylglycindiessigsäure, β-Alanindiessigsäure, Serindiessigsäure, Isoserindiessigsäure, Asparagindiessigsäure, Iminodiessigsäure, Methyliminodiessigsäure, Hydroxyethylethylendiamintriessigsäure, Ethylendiamintetraessigsäure oder Diethylentriaminpentaessigsäure; Phosphonsäuren wie Acetophosphonsäure, Hydroxyphosphonsäuren oder Aminophosphonsäuren, insbesondere Polyphosphonsäuren wie 1-Hydroxyethan-1,1-diphosphonsäure, Aminotri-(methylenphosphonsäure), Alkylendiamintetra-(methylenphosphonsäure) und Dialkylentriaminopenta-(methylenphosphonsäure), insbesondere Ethylendiamintetra-(methylenphosphonsäure), 1,2-Propylendiamintetra-(methylenphosphonsäure) oder 1,3-Propylendiamintetra-(methylenphosphonsäure), 1,2-Butylendiamintetra-(methylenphosphonsäure) oder 1,4-Butylendiamintetra-(methylenphosphonsäure), Diethylendiamintetra-(methylenphosphonsäure), Di-(1,2-Propylen)-diamintetra-(methylenphosphonsäure) oder Di-(1,3-Propylen)-diamintetra-(methylenphosphonsäure) sowie Di-(1,2-Butylen)-diamintetra-(methylenphosphonsäure) oder Di-(1,4-Butylen)-diamintetra-(methylenphosphonsäure); Sulfonsäuren wie Toluolsulfonsäure und Sulfanilsäure; Polyhydroxycarbonsäuren oder Monohydroxycarbonsäuren, insbesondere α-Hydroxycarbonsäuren, wie zum Beispiel Glykolsäure, Milchsäure, Gluconsäure und andere Zuckersäuren, Äpfelsäure, Weinsäure oder Zitronensäure; den Alkalisalzen und Ammoniumsalzen der Monohydroxycarbonsäuren und Polyhydroxycarbonsäuren, insbesondere puffernden Salzen wie Natriumacetat, Kaliumacetat oder Ammoniumacetat, Natriumoxalat, Kaliumoxalat oder Ammoniumoxalat; den Alkalisalzen der obengenannten Phosphonsäuren und Sulfonsäuren; den Alkalisalzen der Hydroxyphosphonsäuren und Aminophosphonsäuren sowie den Alkalisalzen der Aminopolycarbonsäuren; Diammoniumtartrat, Dinatriumtartrat, Dikaliumtartrat oder Kalium/Natriumtartrat; Kaliumhydrogenphthalat, Kaliumhydrogentartrat, Natriumgluconat, Natriumdihydrogencitrat, Kaliumdihydrogencitrat oder Ammoniumdihydrogencitrat, Dinatriumhydrogencitrat, Dikaliumhydrogencitrat oder Diammoniumhydrogencitrat, Trinatriumcitrat oder Trikaliumcitrat sowie Magnesiumcitrat; oder Kombinationen dieser Substanzen.

Als anorganische Säure können beispielsweise genannt werden: Salzsäure, Schwefelsäure oder Phosphorsäure sowie deren Alkalisalze und Ammoniumsalze, insbesondere puffernde Salze wie Natriumhydrogensulfat, Kaliumhydrogensulfat oder Ammoniumhydrogensulfat, Diammoniumsulfat, Natriumdihydrogenphosphat, Kaliumdihydrogenphosphat oder Ammoniumdihydrogenphosphat, Dinatriumhydrogenphosphat, Dikaliumhydrogenphosphat oder Diammoniumhydrogenphosphat sowie Ammoniumchlorid.

Besonders bevorzugt ist hierbei die Verwendung der folgenden Säuren: Oxalsäure, Palmitinsäure, Kaliumhydrogenphthalat und $\alpha$-Hydroxycarbonsäuren, insbesondere Zitronensäure und Weinsäure, oder deren Salze sowie Kombinationen dieser Säuren.

Als geeignete Erdalkaliperoxide können Magnesiumperoxid, Calciumperoxid, Strontiumperoxid oder Bariumperoxid, alleine oder in Kombination miteinander, verwendet werden, wobei die Verwendung von Calciumperoxid und Bariumperoxid besonders bevorzugt ist.

Bezogen auf das gebrauchsfertige Bleichmittel werden die Erdalkaliperoxide in einer Gesamtmenge von 0,1 bis 30 Gewichtsprozent, vorzugsweise in einer Gesamtmenge von 1 bis 20 Gewichtsprozent, insbesondere in einer Gesamtmenge von 5 bis 15 Gewichtsprozent, eingesetzt, während die anorganischen oder organischen Säuren oder deren Salze, bezogen auf das gebrauchsfertige Bleichmittel, in einer Gesamtmenge von 0,1 bis 40 Gewichtsprozent, vorzugsweise in einer Gesamtmenge von 1 bis 30 Gewichtsprozent, insbesondere in einer Gesamtmenge von 5 bis 20 Gewichtsprozent, eingesetzt werden.

Das erfindungsgemäße Bleichmittel enthält vorzugsweise zusätzlich zu den genannten Komponenten (a) und (b) die Bleichwirkung verstärkende Peroxoverbindungen, insbesondere Persulfate, wie zum Beispiel Natriumpersulfat, Kaliumpersulfat, Ammoniumpersulfat oder Mischungen dieser Persulfate, wobei diese Peroxoverbindungen, bezogen auf das gebrauchsfertige Bleichmittel, in einer Gesamtmenge von etwa 10 bis 65 Gewichtsprozent, insbesondere in einer Gesamtmenge von etwa 15 bis 40 Gewichtsprozent eingesetzt werden.

Weitere Bestandteile des erfindungsgemäßen Bleichmittels sind im allgemeinen Tenside und Emulgatoren aus der Gruppe der anionischen, nichtionischen oder ampholytische oberflächenaktiven Verbindungen, beispielsweise Fettalkoholsulfate, Alkansulfonate, Olefinsulfonate, Fettalkoholpolyglykolethersulfate, Alkylpolyglykoside und ethoxylierte Fettalkohole, Fettsäuren, Alkylphenole, Sorbitanfettsäureester und Fettsäurealkanolamide; Verdicker und Gelbildner wie zum Beispiel Fettalkohole, Fettsäuren, Paraffinöle, Fettsäureester, Methylcellulose oder Hydroxyethylcellulose, Stärke, synthetische Polymerisate wie Polyvinylpyrrolidon und Polyacrylate, oder Biopolymere wie Alginsäure; Stabilisatoren für Peroxoverbindungen wie zum Beispiel Silikate; sowie Komplexbildner; Parfümöle und haarpflegende Zusätze wie kationische Polymere, Lanolinderivate, Cholesterin, Pantothensäure, Proteinderivate und Proteinhydrolysate, Provitamine und Vitamine, sowie Pflanzenextrakte. Diese Zusätze werden zur Herstellung des erfindungsgemäßen Bleichmittels in für diesen Zweck üblichen Mengen eingesetzt; beispielsweise die Tenside und Emulgatoren in einer Konzentrationen von 0,2 bis 30 Gewichtsprozent und die Verdickungsmittel in einer Konzentrationen von 0,1 bis 25 Gewichtsprozent (jeweils bezogen auf das gebrauchsfertige Bleichmittel).

Der pH-Wert des gebrauchsfertigen Bleichmittels beträgt in der Regel etwa 7 bis 11,5, wobei ein pH-Wert von etwa 8 bis 10,5 und insbesondere von 8,5 bis 10 bevorzugt ist.

Wegen der Hydrolyseempfindlichkeit können die Erdalkaliperoxide nicht in wäßriger Lösung aufbewahrt werden. Das erfindungsgemäße Bleichmittel ist daher wasserfrei, wobei der Begriff „wasserfrei" so zu verstehen ist, daß das in den verwendeten Rohstoffen enthaltene Kristallwasser beziehungsweise die in den verwendeten Rohstoffen enthaltenen Feuchtigkeitsspuren in dem erfindungsgemäßen Bleichmittel (bezogen auf das gebrauchsfertige Bleichmittel) in einer Gesamtmenge von maximal 10 Gewichtsprozent, vorzugsweise in einer Gesamtmenge von weniger als 5 Gewichtsprozent, insbesondere in einer Gesamtmenge von weniger als 3 Gewichtsprozent, enthalten ist.

Das erfindungsgemäße Oxidationssystem aus Erdalkaliperoxid und organischer oder anorganischer Säure wird als Gemisch mit den übrigen, üblicherweise in Bleichmitteln enthaltenen Komponenten als wasserfreies Pulver oder in einem wasserfreien flüssigen oder cremeartigen Medium („Bleichpaste/Blondierpaste") als kosmetische Zubereitung formuliert. Im Falle der Pulverformulierung wird in der Regel eine Entstaubung durch Besprühen mit inerten Trägermaterialien wie zum Beispiel Paraffinölen, Siliconölen, Polyethern, Polyorganosiloxanen (beispielsweise den im International Cosmetic Ingredient Dictionary, 5th Ed., Seite 220/221 (1993) unter der Bezeichnung „Dimethicone" aufgeführten Dimethylpolysiloxanen) oder Wachsen durchgeführt.

Je nach verwendeter organischer oder anorganischer Säure kann die Säure auch in Form einer wäßrigen Lösung vorliegen, welche getrennt von der wasserfreien, die Erdalkaliperoxide enthaltenden Zubereitung aufbewahrt wird und erst kurz vor der Anwendung mit den Erdalkaliperoxiden vermischt wird. Ebenfalls ist es möglich, daß das Bleichpulver beziehungsweise das Erdalkaliperoxid in mikroverkapselter Form vorliegt beziehungsweise in einer wasserlöslichen Umhüllung (beispielsweise einem Beute aus Polyvinylalkohol oder Polyvinylpyrrolidon) abgepackt wird und erst beim Vermischen mit Wasser beziehungsweise einer wäßrigen Lösung der organischen oder anorganischen Säure freigesetzt wird.

Die Anwendung des erfindungsgemäßen Bleichmittels erfolgt, indem man unmittelbar vor dem Gebrauch eine für die Haarblondierung/Haarbleichung ausreichende Menge, vorzugsweise 20 bis 70 Gramm, des erfndungsgemäßen Bleichmittels mit Wasser in einem Gewichtsverhältnis von 2:1 bis 1:5, vorzugsweise 1:1 bis 1:3, vermischt, wobei die Vermischung in einer Schale oder in einer geeigneten Anschüttelflasche erfolgen kann. Es wird eine (bei Verwendung von Verdickern angedickte) Blondiermasse/Bleichmittelmasse erhalten, die sich problemlos auf das Haar auftragen läßt, ohne während der Einwirkungszeit abzulaufen.

Prinzipiell ist es zwar möglich, dem erfindungsgemäßen Bleichmittel zusätzlich geringe Mengen an Wasserstoffperoxid (maximal 2 Gewichtsprozent, bezogen auf das gebrauchsfertige Bleichmittel) zuzusetzen; vorzugsweise wird das erfindungsgemäße Bleichmittel jedoch ohne den Zusatz von Wasserstoffperoxid verwendet.

Die Anwendungstemperatur liegt in einem Bereich von etwa 10 bis 50°C, vorzugsweise 20 bis 40°C, wobei der Bleichvorgang durch das Einwirken von wärme während der Behandlung beschleunigt werden kann. Nach einer Einwirkungszeit von 10 bis 80 Minuten, vorzugsweise 30 bis 60 Minuten, insbesondere 45 Minuten bei 40°C und 45 bis 60 Minuten bei 20°C, wird die Blondiermasse/Bleichmittelmasse mit Wasser gründlich ausgespült, das Haar gegebenenfalls mit einem Shampoo gewaschen und sodann getrocknet.

Das erfindungsgemäße Bleichmittel ermöglicht ohne die Zugabe von Wasserstoffperoxid eine äußerst milde und schonende Bleichung/Blondierung der Haare mit hervorragenden Bleichergebnissen.

Die folgenden Beispiele sollen den Gegenstand der Erfindung näher erläutern, ohne diesen hierauf zu beschränken.

**Beispiele**

**Beispiel 1:** Blondiermittel

| | |
|---|---|
| 7,9 g | Calciumperoxid |
| 20,5 g | Kaliumperoxodisulfat |
| 12,4 g | Ammoniumperoxodisulfat |
| 8,2 g | Natriumperoxodisulfat |
| 43,2 g | Dinatriumcitrat |
| 3,0 g | Calciumhydroxid |
| 2,1 g | Natriumalginat |
| 1,5 g | Calciumcarbonat |
| 0,4 g | Dinatrium-Ethylendiaminotetraacetat |
| 0,4 g | Siliciumdioxid |
| 0,4 g | Natriumcocoylisethionat |
| 100,0 g | |

50 g des vorstehenden Blondiermittels werden unmittelbar vor dem Gebrauch mit 75 g Wasser innig vermischt. Die erhaltene gebrauchsfertige Blondiermasse läßt man 45 Minuten bei 40°C auf dunkle Naturhaare einwirken. Anschließend wird das Haar gründlich mit Wasser ausgespült, mit einem Shampoo gewaschen und sodann getrocknet. Das Haar ist intensiv und gleichmäßig aufgehellt.

**Beispiel 2:** Blondiermittel

| | |
|---|---|
| 11,6 g | Magnesiumperoxid |
| 42,8 g | Ammoniumperoxodisulfat |
| 14,3 g | Magnesiumcitrat |
| 31,3 g | Magnesiumoxid |
| 100,0 g | |

50 g des vorgenannten Bleichmittels werden unmittelbar vor dem Gebrauch mit 50 g einer 14prozentigen wäßrigen Zitronensäurelösung innig vermischt. Die so erhaltene Blondiermasse läßt man 45 Minuten bei 40°C auf dunkle Naturhaare einwirken. Anschließend wird das Haar gründlich mit Wasser ausgespült, mit einem Shampoo gewaschen und abschließend getrocknet. Das Haar ist intensiv und gleichmäßig aufgehellt.

**Beispiel 3:** Haarbleichmittel

| | |
|---|---|
| 21,5 g | Bariumperoxid |
| 23,8 g | Kaliumperoxodisulfat |
| 9,6 g | Natriumperoxodisulfat |
| 15,3 g | Palmitinsäure |
| 10,5 g | Natriumoxalat |
| 0,5 g | Dinatrium-EDTA |
| 0,5 g | Siliciumdioxid |
| 0,5 g | Natriumcocoylisethionat |
| 3,4 g | Cellulose Gum |
| 100,0 g | |

50 g des vorgenannten Bleichmittels werden kurz vor dem Gebrauch mit 75 g Wasser innig vermischt. Das so erhaltene Haarbleichmittel läßt man 45 Minuten bei 40°C auf dunkle Naturhaare einwirken. Anschließend wird das Haar gründlich mit Wasser ausgespült, mit einem Shampoo gewaschen und sodann getrocknet. Das Haar ist intensiv und gleichmäßig aufgehellt.

Alle in der vorliegenden Anmeldung genannten Prozentzahlen stellen, soweit nicht anders angegeben, Gewichtsprozente dar.

**Patentansprüche**

1. Mittel zum Entfärben oder Blondieren von Keratinfasern, insbesondere menschlichen Haaren, welches a) mindestens ein Erdalkaliperoxid und b) mindestens eine organische oder anorganische Säure enthält.

2. Mittel nach Anspruch 1, dadurch gekennzeichnet, daß das Erdalkaliperoxid ausgewählt ist aus Magnesiumperoxid, Calciumperoxid, Bariumperoxid und Strontiumperoxid sowie Kombinationen dieser Verbindungen.

3. Mittel nach Anspruch 1 oder 2, dadurch gekennzeichnet, daß es das Erdalkaliperoxid in einer Menge von 0,1 bis 30 Gewichtsprozent enthält.

4. Mittel nach einem der Ansprüche 1 bis 3, dadurch gekennzeichnet, daß die Säure ausgewählt ist aus Oxalsäure, Palmitinsäure, Kaliumhydrogenphthalat und α-Hydroxycarbonsäuren oder deren Salze sowie Kombinationen dieser Säuren.

5. Mittel nach Anspruch 4, dadurch gekennzeichnet, daß die α-Hydroxycarbonsaure ausgewählt iß aus Weinsäure und Zitronensäure.

6. Mittel nach einem der Ansprüche 1 bis 5, dadurch gekennzeichnet, daß es die organische oder anorganische Säure in einer Menge von 0,1 bis 40 Gewichtsprozent enthält.

7. Mittel nach einem der Ansprüche 1 bis 6, dadurch gekennzeichnet, daß es mindestens eine die Bleichwirkung verstärkende Peroxoverbindung enthält.

8. Mittel nach Anspruch 7, dadurch gekennzeichnet, daß die Peroxoverbindung ausgewählt ist aus Natriumpersulfat, Kaliumpersulfat, Ammoniumpersulfat und Mischungen dieser Persulfate.

9. Mittel nach einem der Ansprüche 1 bis 8, dadurch gekennzeichnet, daß es die die Bleichwirkung verstärkende Peroxoverbindung in einer Menge von 10 bis 65 Gewichtsprozent enthält.

10. Mittel nach einem der Ansprüche 1 bis 9, dadurch gekennzeichnet, daß die gebrauchsfertige Zubereitung einen pH-Wert von 7 bis 11,5 aufweist.

11. Verfahren zum Entfärben oder Blondieren von Haaren, dadurch gekennzeichnet, daß 20 bis 70 g eines Mittels nach einem der Ansprüche 1 bis 10 mit Wasser in einem Verhältnis ( Mittel:Wasser) von 2 : 1 bis 1 : 5 vermischt werden, anschließend die so erhaltene Bleichmittelmasse auf das Haar aufgetragen wird, das Haar nach einer Einwirkungszeit von 10 bis 80 Minuten bei 10 bis 50°C mit Wasser ausgespült und gegebenenfalls mit einem Shampoo gewaschen wird und abschließend getrocknet wird.

12. Verfahren nach Anspruch 11, dadurch gekennzeichnet, daß anstelle von Wasser eine wäßrige Lösung einer organischen oder anorganischen Säure verwendet wird.

| Europäisches Patentamt | EUROPÄISCHER RECHERCHENBERICHT | Nummer der Anmeldung<br>EP 97 11 8999 |

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| E | EP 0 815 830 A (GOLDWELL GMBH) 7.Januar 1998<br>* das ganze Dokument *<br>--- | 1-3 | A61K7/135 |
| X | GB 859 276 A (UNILEVER LIMITED)<br><br>* das ganze Dokument *<br>--- | 1-4,10, 11 | |
| P,X | DE 196 00 704 A (HENKEL KGAA) 17.Juli 1997<br>* Seite 1, Zeile 40 - Seite 4, Zeile 34; Anspruch 1 *<br>--- | 1-4 | |
| A | DE 44 31 577 A (WELLA AG) 7.März 1996<br>* Seite 1, Zeile 40-41 *<br>--- | 1 | |
| A | EP 0 574 696 A (GOLDWELL AG) 22.Dezember 1993<br>* das ganze Dokument *<br>----- | 1,2 | |

RECHERCHIERTE SACHGEBIETE (Int.Cl.6)

A61K

Der vorliegende Recherchenbericht wurde für alle Patentansprüche erstellt

| Recherchenort<br>DEN HAAG | Abschlußdatum der Recherche<br>19.Juni 1998 | Prüfer<br>Couckuyt, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)